Europäisches Patentamt

European Patent Office

Office européen des brevets

(19)

(11) Numéro de publication: **0 332 519**
**A1**

## (12) DEMANDE DE BREVET EUROPEEN

(21) Numéro de dépôt: 89400609.7

(22) Date de dépôt: 03.03.89

(51) Int. Cl.⁴: **A 61 B 6/00**

(30) Priorité: 08.03.88 FR 8802916

(43) Date de publication de la demande:
13.09.89 Bulletin 89/37

(84) Etats contractants désignés: **DE ES GB IT NL**

(71) Demandeur: **GENERAL ELECTRIC CGR SA**
**100, rue Camille Desmoulins**
**F-92137 ISSY LES MOULINEAUX (FR)**

(72) Inventeur: **Lieutaud, Olivier**
**Cabinet Ballot-Schmit 84, Avenue Kléber**
**F-75116 Paris (FR)**

**Marie, Alain**
**Cabinet Ballot-Schmit 84, Avenue Kléber**
**F-75116 Paris (FR)**

(74) Mandataire: **Ballot, Paul Denis Jacques**
**Cabinet Ballot-Schmit 84, avenue Kléber**
**F-75116 Paris (FR)**

(54) Mamographe.

(57) On réalise un mamographe dans lequel le tube (2) à rayons X et le plan (1) de travail sont maintenus en vis à vis l'un de l'autre par un étrier (21,22) permettant un accès aisé d'une opératrice sur le sein à radiographier. L'étrier (22) a une forme de couronne circulaire et est susceptible de tourner sur lui-même en coulissant sur une couronne (21) complémentaire de manière à réduire l'inertie des masses déplaçables pour provoquer une radiographie sous une incidence oblique ou même horizontale. En outre, la structure est de préférence déportée (16) d'une armoire (18) de contrôle du mamographe pour permettre à une opératrice de s'interposer entre cette armoire et la patiente, en vue de faciliter la préparation de la prise du cliché.

FIG_1

EP 0 332 519 A1

**Description**

## MAMOGRAPHE

La présente invention a pour objet un mamographe, dit aussi senographe, utilisable en radiologie et plus particulièrement dans le domaine médical. Un mamographe est un appareil permettant de donner une image des structures tissulaires d'un sein d'une patiente sous examen. L'invention a pour but de faciliter les interventions des opérateurs sur de tels appareils, de donner en conséquence plus de confort à la patiente qui subit un examen de radiographie du sein avec un tel appareil.

Les mamographes comportent pour l'essentiel un tube à rayons X et un plan de travail. Le rayonnement des rayons X du tube est dirigé vers le plan de travail, la plupart du temps sensiblement perpendiculairement à ce plan. Le plan de travail comporte une surface destinée à recevoir en appui le sein à radiographier. En aval de cette surface par rapport au rayonnement, un logement est aménagé sous ce plan. Il est destiné à recevoir une cassette munie d'un film radiosensible pour rendre compte de l'examen radiographique. Eventuellement un plateau d'appui, ou pelote, transparent au rayonnement X, est interposé entre le foyer du tube et le plan de travail pour venir comprimer le sein sous examen au moment de la prise du cliché. Du fait de cette compression, le plateau est en outre muni de dispositifs pour relâcher la pression dès que le cliché a été pris. Le réglage d'un mamographe est déterminé en usine en ce qui concerne la distance séparant le foyer du tube à rayons X (en fait le tube à rayons X lui-même) et la surface du plan de travail. Cette distance est en principe fixe. Pour pouvoir tenir compte des différences de taille des patientes ces deux équipements sont montés fixement en porte à faux sur un mât. Le mât peut alors s'élever ou s'abaisser par rapport à une position moyenne pour tenir compte de cette différence de taille des patientes. En outre, pour certains examens, il est utile de pratiquer une irradiation latérale du sein à radiographier. Aussi, le mât, qui supporte le tube à rayons X et le plan de travail, est-il solidaire d'un arbre horizontal. Cet ensemble peut alors tourner autour de cet arbre. Par exemple, on bascule le mât d'un quart de tour autour de l'arbre de manière à obtenir un alignement horizontal du tube et du plan de travail.

Ces mamographes présentent en pratique de nombreux inconvénients. En effet, il est connu que l'alignement de l'arbre de rotation passe sensiblement à mi-hauteur de la distance qui sépare le foyer du tube à rayons X du plan de travail. En pratique il peut même être placé le plus près possible du plan de travail lui-même, de manière à ne pas avoir à effectuer de correction de hauteur du dispositif au moment de la prise des clichés latéraux. En conséquence, au moment de la prise de ces clichés latéraux, le tube à rayons X se trouve doublement en porte à faux par rapport à l'appareil. Il se trouve d'une part en porte à faux vers l'avant de l'appareil, vers l'avant du mât, comme on a pu le constater précédemment. Et il se trouve aussi d'autre part maintenant en porte à faux par rapport à l'arbre. Or le tube à rayons X avec son équipement de commande et d'isolation est lourd. Il entraîne naturellement la rotation de l'arbre, le basculement du mât. Pour éviter d'avoir à installer des freins de limitation du basculement du mât lorsque le tube est en prise de cliché latéral, on a l'habitude de placer un contre-poids en bout de mât sensiblement symétriquement au tube à rayons X par rapport à l'arbre. Ce contre-poids présente deux inconvénients. D'une part il est encombrant. En effet étant relevé au moment des prises de clichés latéraux : il gêne le passage de l'opérateur. D'autre part il contribue à rendre la manipulation de l'ensemble tube à rayons X-plan de travail difficile au moment du changement d'incidence. En effet l'inertie présentée par un tel dispositif est grande. Comme la manipulation de l'ensemble est manuelle, cette inertie est trop grande pour permettre des grandes cadences de travail, pour permettre le passage d'un nombre important de patientes devant l'appareil pendant une durée donnée.

En outre, l'examen radiographique d'un sein peut être fait aussi avec l'injection d'un produit de contraste. Par exemple, on injecte un produit opaque aux rayonnements X par le mamelon du sein, par les canaux galactophores, juste au moment de prendre le cliché. La présence du mât, situé en face directe du sein, gêne alors cette manoeuvre tout en constituant par ailleurs une gêne pour la patiente. Au moment de cette manoeuvre délicate, celle-ci peut rechercher un contact visuel avec l'opératrice, ce qui lui est interdit par la présence du mât qui s'interpose entre elles.

On connait en outre d'autres structures de maintien où le mât a été remplacé par un étrier suspendu au plafond. Le tube à rayons X est maintenu au sommet de l'étrier. Le plan de travail est accroché, par ses deux bords latéraux, aux extrémités des jambes de l'étrier. Dans ce cas l'accès à la patiente est aisé. Mais la manipulation de la suspension pour l'amener dans des orientations correspondant à des incidences latérales ou obliques ne peut plus alors être que motorisée. Elle est en conséquence lente, l'équipement est quant à lui coûteux.

L'invention a pour objet de remédier à ces inconvénients en proposant une structure de maintien respectif du tube à rayons X et du plan de travail qui soit mieux adaptée à sa manipulation au moment des changements d'incidence et qui permette un bon contact technique et psychologique entre la patiente et l'opératrice. En substance, l'invention prévoit de remplacer l'étrier par une couronne circulaire. Au sommet de la couronne circulaire est accroché le tube à rayons X . Des extrémités d'arcs de la couronne sont alors fixées latéralement au plan de travail. Il en résulte que dans l'arche formé à l'intérieur de cet étrier circulaire, et qui en quelque sorte surplombe le plan de travail, il est maintenant possible d'accéder au sein de la patiente alors que celle-ci peut voir l'opératrice. En outre la manipulation du tube dont le point de fixation coulisse sur le pourtour de la couronne est alors grandement facilitée. On peut facilement changer d'incidence sans se heurter à une grande inertie. Il n'y a plus de contrepoids gênant. Enfin la couronne circulaire peut être fixée a un mât où a une console déplaçable verticalement de manière à s'adapter aux tailles des patientes.

De manière à faciliter encore le travail de l'opératrice, l'étrier circulaire est suspendu à une potence qui écarte cet ensemble du mât. L'opératrice peut alors se placer sous cette potence, entre la patiente et l'armoire contenant tous les organes de contrôle de l'appareil.

L'invention a donc pour objet un mamographe comportant un plan de travail, un tube à rayons X orienté vers ce plan de travail, et une structure de maintien du tube à rayons X par rapport au plan de travail comportant une partie terminale en forme d'étrier, pour maintenir un tube à rayons X d'une part, et pour maintenir par les extrémités des jambes de l'étrier les bords latéraux du plan de travail d'autre part caractérisé en ce que cette partie terminale de la structure est en forme de couronne circulaire.

L'invention sera mieux comprise à la lecture de la description qui suit et à l'examen des figures qui l'accompagnent. Celles-ci ne sont données qu'à titre indicatif et nullement limitatif de l'invention. Les figures montrent :

- figure 1 : un mamographe muni d'un étrier circulaire selon l'invention ;
- figure 2 : une variante de la réalisation précédente dans laquelle une potence est remplacée par une console latérale.

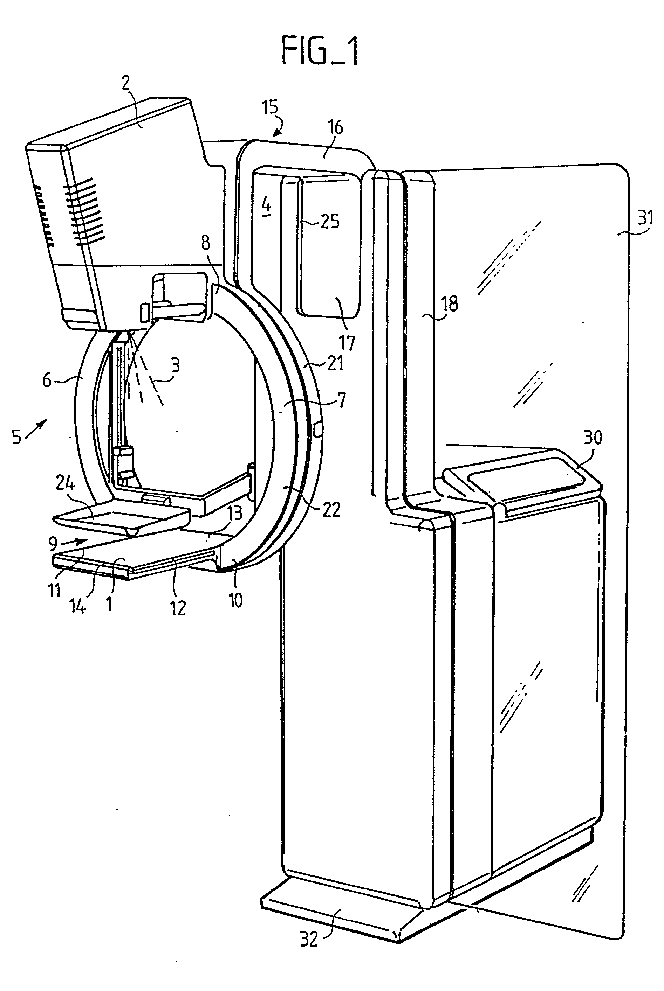

La figure 1 montre un mamographe comportant un plan de travail 1 et un tube à rayons X 2 dont le rayonnement X 3 est orienté vers le plan de travail 1. Une structure 4 permet de maintenir d'une manière fixe, réglée en usine, respectivement l'un par rapport à l'autre, le plan de travail 1 et le tube à rayons X 2. La structure 4 comporte essentiellement un étrier circulaire 5 muni de jambes, ou d'arcs de couronne circulaire respectivement 6 et 7, et d'un sommet 8. Le tube à rayons X est fixé au sommet 8 de l'étrier. Les extrémités 9 et 10 respectivement des jambes 6 et 7 sont fixées aux bords latéraux, respectivement 11 et 12 en correspondance du plan de travail 1. Dans un exemple, la surface du plan de travail a une forme sensiblement rectangulaire, et les extrémités 9 et 10 des jambes 6 et 7 sont fixées sur les bords 11 et 12 du plan de travail, à proximité d'un bord 13 de ce plan, bord 13 lui même opposé à un bord 14 contre lequel la patiente vient se mettre en appui. Le plan 1 est transparent au rayonnement X. Il comporte sur les bords 11 et 12 des glissières pour venir fixer une cassette, non représentée, comportant un film radiosensible.

La structure 4 comporte également une potence 15 au sommet de laquelle est pendu solidairement le sommet 8 de l'étrier circulaire 5. Cette potence 15 comporte une poutre en porte à faux 16 et un mât 17. La longueur de la poutre 16 permet d'éloigner l'étrier 5 et tous les équipements qu'il supporte d'une armoire de contrôle 18 du mamographe. Cet écart permet alors à l'opératrice de s'interposer entre l'armoire 18 et l'étrier 5 pour intervenir sur le sein à radiographier. Du fait de l'écart qui permet le passage de l'opératrice, le poids de l'armoire 18 doit être suffisant pour empêcher que le mamographe en entier ne bascule vers l'avant.

Pour permettre les incidences obliques ou même horizontales, l'étrier a maintenant une forme circulaire et il se compose d'au moins deux couronnes. Une première couronne 21 est par exemple solidaire de la potence 15 tandis qu'une seconde couronne 22 supporte le tube à rayons X d'une part, et le plan de travail 1 d'autre part. Les couronnes 21 et 22 peuvent tourner l'une sur l'autre à la manière des deux couronnes d'un roulement à billes. Dans un exemple , chaque couronne comporte une glissière dont le profil est complémentaire de celui de l'autre glissière, ou de celui d'une troisième couronne intermédiaire entre ces deux couronnes, de manière à ce qu'elles s'agrippent l'une sur l'autre, et de manière à ce qu'elles ne puissent plus autoriser comme mouvement relatif que des rotations autour de leur axe de rotation commun.

Cet axe de rotation des couronnes est placé, sensiblement à mi-hauteur entre le centre de gravité du tube à rayons X et la surface d'appui du plan de travail. Pour ne pas avoir à effectuer de réglages importants de hauteur au moment du passage en incidence, la surface du plan de travail 1 pourrait être placée sensiblement légèrement en dessous de l'alignement de cet axe de rotation. Il suffirait de remonter en conséquence la fixation du plan de travail 1 par rapport au centre de rotation de la couronne circulaire. Si les besoins s'en faisait sentir le plan de travail pourrait aussi être décalé légèrement vers le bas de la couronne. La solution de l'invention apporte une amélioration notable par rapport à l'état de la technique en ce sens que l'inertie opposée au moment du changement d'incidence se réduit à l'inertie résultant de la masse du tube à rayons X, de la masse du plan de travail, et de la masse de l'étrier, sans plus avoir à prendre en compte des masses d'un mât basculant, des masses de poutre, et des masses de contre-poids. La présence de la potence 15 permet néanmoins comme précédemment à l'opératrice d'intervenir facilement en s'insérant entre l'armoire 18 et la patiente.

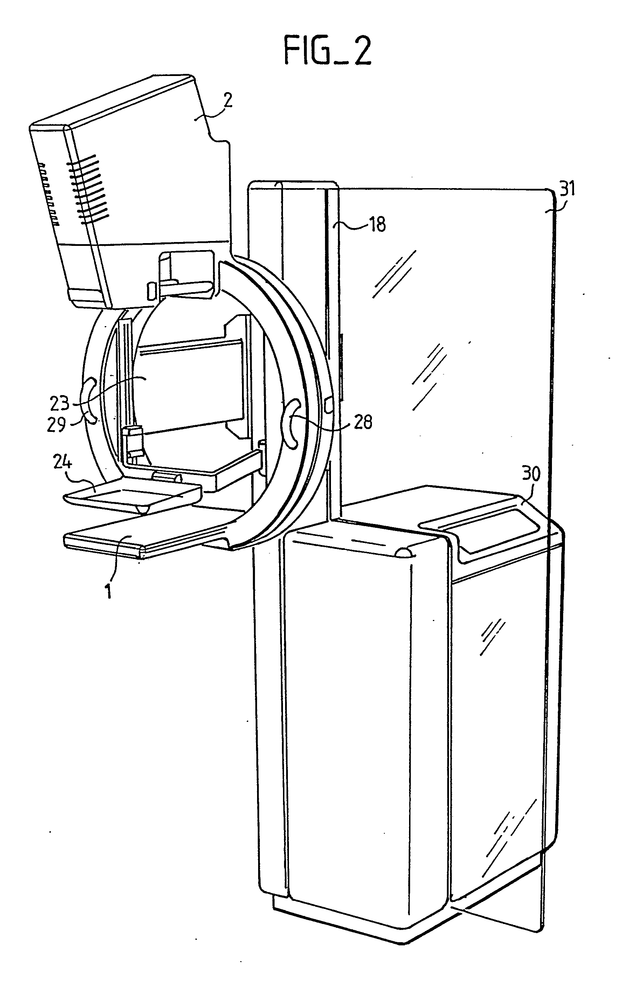

La figure 2 montre une variante de la figure 1 dans laquelle, la potence munie de son mât vertical est remplacée par une console 23 sensiblement horizontale. La console 23 vient soutenir une des jambes , par exemple la jambe 6 de l'étrier 5. Sur cette figure 2 l'étrier est aussi un étrier de forme circulaire permettant également la réduction de l'inertie au moment du changement d'incidence. La console 23 peut éventuellement être télescopique et coulisser dans une glissière 33 fixée à l'armoire 18.

Pour tenir compte des différences de taille des patientes, la structure peut bien entendu se déplacer verticalement. Sur la figure 1 le mât 17 se déplace verticalement. Compte tenu du poids, ce déplacement doit être motorisé. Le déplacement du mât est souvent équilibré par des contre-poids reliés par des chaînes et des paliers au mât 19. Le déplacement du mât est guidée par le frottement de galets dans des guides tels que le fourreau 25. On remarque que le déplacement vertical de la structure 4 de la figure 1 est plus simple que le déplacement vertical d'un arbre de basculement d'un mât de l'état de la technique. En effet, il est plus simple de déplacer longitudinalement un mât que de déplacer un mât et un arbre de rotation. En pratique, compte tenu du contre-poids évitant le basculement du mamographe sur l'avant, l'arbre de rotation doit s'enfoncer suffisamment profondément dans l'armoire 18. Pendant le déplacement vertical, cet arbre balaye un espace, bordé par les guides, à l'intérieur de cette armoire. Cet espace ne peut pas être occupé par les appareils de contrôle du mamographe. Le mamographe doit alors être plus gros.

Pour certains hôpitaux où des problèmes d'encombrement se présentent, la solution avec la console horizontale de la figure 2 s'impose. Celle-ci limite cependant l'accès de l'opératrice par un seul côté dans l'espace ménagé entre la patiente et les armoires de contrôle. Par contre cette solution moins lourde ne nécessite pas la présence d'un sabot 32 (figure 1) en pied de l'armoire 18 pour équilibrer le porte-à-faux de l'ensemble tube-plan de travail.

Pour la mise en compression du sein lors d'un examen radiographique, on utilise un plateau d'appui ou pelote 24. De préférence ce plateau d'appui est également maintenu par ses bords latéraux sur les jambes respectivement 6 et 7 de l'étrier 5. Le mécanisme de commande de compression et de relâchement de ce plateau peut-être réglé de manière à relâcher simultanément les deux appuis au moment de la fin du cliché. Ce mécanisme est d'un type connu.

Pour la manipulation de l'étrier enfin celui ci peut être muni de poignées latérales comme les poignées 28 et 29 de la figure 2 par exemple. Le mamographe est encore muni d'autres moyens classiques tels qu'un pupitre 30 de commande, ou une vitre 31 de protection des opératrices.

L'invention apporte en outre un autre avantage. Il est possible de maintenir le plan de travail fixe tandis que l'on fait tourner le tube à rayons X seul. Dans ce cas ce tube et le plan de travail ne sont pas solidaires d'une seule est même couronne. Ils sont solidaires chacun d'une couronnes différente. Ces couronnes peuvent être maintenues, par une troisième couronne, imbriquées entre elles. On peut alors réaliser des incidences obliques d'un type particulier. On peut modifier les conditions de réglage en usine de la position du tube par rapport au plan de travail.

## Revendications

1 - Mamographe comportant un plan (1) de travail, un tube (2) à rayons X orienté (3) vers ce plan de travail, et une structure (4) de maintien du tube à rayons X par rapport au plan de travail, la structure comportant un étrier (5) pour maintenir le tube à rayons X d'une part, et pour maintenir par les extrémités (9,10) des jambes (6,7) de l'étrier les bords latéraux (11,12) du plan de travail d'autre part caractérisé en ce que cet étrier est en forme générale de couronne circulaire (21, 22) et qu'il comporte, pour recevoir en coulissement circulaire le tube par rapport au plan de travail, une couronne (21) de maintien pour maintenir le plan de travail qui peut coulisser dans une couronne de fixation (22) pour maintenir le tube.

2 - Mamographe selon la revendication 1 caractérisé en ce que la structure comporte des moyens (16) pour écarter la partie terminale de la structure d'une armoire (18) de contrôle de ce mamographe.

3 - Mamographe selon l'une quelconque des revendications 1 à 2 caractérisé en ce que la structure comporte une potence (15)

4 - Mamographe selon l'une quelconque des revendications 1 à 3 caractérisé en ce que la structure comporte une console latérale (23).

5 - Mamographe selon l'une quelconque des revendications 1 à 4 caractérisé en ce que la structure est déplaçable (25) verticalement.

6 - Mamographe selon l'une quelconque des revendications 1 à 5 comportant un plateau d'appui (24) pour maintenir en compression les seins à examiner, caractérisé en ce que le plateau est maintenu par ses bords latéraux sur les deux jambes de la couronne de maintien de l'étrier.

# FIG_1

## FIG_2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 363 316  (SIEMENS)<br>* Page 1, lignes 1-9; page 4, ligne 23 - page 5, ligne 38; figure 1 * | 1 | A 61 B   6/00 |
| A | | 4,5 | |
| Y | DE-A-3 321 057  (P. RÖDER)<br>* Résumé; page 5, lignes 5-14; figure 1 * | 1 | |
| A | DE-C-  973 092  (C.H.F. MÜLLER)<br>* Page 2, lignes 39-59; figure 1 * | 1,5 | |
| A | US-A-3 824 397   (M. BAUER et al.)<br>* En entier * | 1,2,6 | |
| A | EP-A-0 229 971  (SIEMENS)<br>* Page 3, lignes 10-23; figure 1 * | 1-3,5 | |

DOMAINES TECHNIQUES
RECHERCHES (Int. Cl.4)

A 61 B

Le présent rapport a été établi pour toutes les revendications

| Lieu de la recherche | Date d'achèvement de la recherche | Examinateur |
|---|---|---|
| LA HAYE | 05-06-1989 | FERRIGNO, A. |

EPO FORM 1503 03.82 (P0402)